# EUROPÄISCHE PATENTSCHRIFT

(11) **EP 3 213 700 B1**
(45) Veröffentlichungstag und Bekanntmachung des Hinweises auf die Patenterteilung: **24.10.2018**
(21) Anmeldenummer: 17155947.9
(22) Anmeldetag: 14.02.2017
(51) Int. Cl.: A61B 17/29, A61B 17/062

(54) **MEDIZINISCHES INSTRUMENT**
MEDICAL INSTRUMENT
INSTRUMENT MÉDICAL

(30) Priorität: 01.03.2016 DE 102016103640
(43) Veröffentlichungstag der Anmeldung: 06.09.2017
(73) Patentinhaber: Karl Storz SE & Co. KG, 78532 Tuttlingen (DE)
(72) Erfinder: Kärcher, Daniel, 78532 Tuttlingen (DE); Stefan, Jochen, 78532 Tuttlingen (DE)

(56) Entgegenhaltungen:
- EP-A1- 2 522 280
- US-A- 5 626 607
- US-B2- 7 896 900

## Beschreibung

Die vorliegende Erfindung betrifft ein medizinisches Instrument, insbesondere ein chirurgisches Instrument, mit einem langerstreckten Schaft und einem an einem distalen Endabschnitt des Schafts angeordneten Werkzeug, das zwei insbesondere zum Halten eines Gegenstands miteinander zusammenwirkende Werkzeugelemente umfasst, von denen mindestens eines mittels eines längsverschiebbaren ersten Übertragungselements bewegbar ist.

Bei derartigen medizinischen Instrumenten stellt sich häufig die Aufgabe, dass ein Gegenstand mit dem Werkzeug gefasst und fest gehalten werden soll, um den Gegenstand mittels des chirurgischen Instruments bewegen und beispielsweise chirurgische Manipulationen mit dem Gegenstand ausführen zu können. So soll beispielsweise eine chirurgische Nadel gefasst und möglichst fest gehalten werden, um die Nadel in Gewebe einzustechen. Hierfür ist es wünschenswert, die miteinander zusammenwirkenden Werkzeugelemente in einer Halteposition, in der das chirurgische Element gegriffen ist, zu blockieren bzw. zu verriegeln, so dass ein Benutzer zum weiteren Halten des Gegenstands keine Kraft mehr aufwenden muss und diesen bequem bewegen kann. Eine derartige Verriegelung kann beispielsweise mittels einer an einer Handhabe angeordneten Raste erfolgen, mit der ein Handgriff, mit dem über eine Zugstange das mindestens eine bewegbare Werkzeugelement betätigt werden kann, in einer Halteposition verriegelt werden kann. Auf diese Weise kann zwar ein sicheres und festes Halten des Gegenstands ermöglicht werden, ohne dass hierfür eine fortgesetzte Kraftaufwendung durch den Benutzer erforderlich ist. Allerdings steht dabei die Zugstange unter Spannung, was im Hinblick auf die Handhabung und die Haltbarkeit des Instruments nachteilig ist. Ferner steht in dem Fall, dass das Werkzeug um eine Längsachse drehbar gelagert ist, im verriegelten Zustand das Drehlager des Werkzeugs unter Spannung und kann nur mit erheblichem Kraftaufwand rotiert werden. Die hohe Belastung bei Rotation kann auch zu einer Beschädigung des Instruments führen.

Aus DE 10 2009 055 747 A1 ist eine chirurgische Zange bekannt, wobei eine Relativbewegung der Maulteile des Zangenmauls über den Eingriff wenigstens eines Nockens am Zangenmaul oder an einer Betätigungsstange in einer schräg zur Verschiebungsrichtung der Betätigungsstange an dieser oder am Zangenmaul angeordneten Nut gesteuert wird. Die Nut ist wenigstens bereichsweise mit einem Neigungswinkel ausgebildet, der kleiner ist als der Neigungswinkel, bis zu dem Selbsthemmung in der Nut besteht. Die Selbsthemmung sorgt dafür, dass nach Festklemmen eines Gegenstands im Zangenmaul durch Betätigen der Betätigungsstange die Klemmstellung aufrechterhalten bleibt, auch wenn die Betätigungsstange losgelassen wird.

In EP 2 522 280 A1 ist ein medizinisches Instrument offenbart, insbesondere ein chirurgischer Nadelhalter, der zwei Maulteile umfasst, wobei mindestens eines der Maulteile relativ zu dem anderen zwischen einem Greifzustand zum Greifen des Objekts und einem Freigebezustand zum Freigeben des Objekts schwenkbar ist. Das Instrument weist einen Riegelmechanismus zum Blockieren des mindestens einen schwenkbaren Maulteils im Greifzustand auf, wobei ein Verbindungsstift, der das schwenkbare Maulteil mit einem Riegelelement verbindet, in einer länglichen Öffnung des Riegelelements aufgenommen ist. Die längliche Öffnung ist durch periodische Vorsprünge in mehrere Abschnitte aufgeteilt, so dass das schwenkbare Maulteil stufenweise aus einer stabilen verriegelten Position in eine andere stabile verriegelte Position schwenkbar ist.

Gemäß US 7,896,900 B2 ist bei einem medizinischen Instrument, insbesondere einem chirurgischen Nadelhalter, ein Riegelmechanismus zum Feststellen mindestens eines schwenkbaren Maulteils in einem Greifzustand zum Greifen eines Objekts vorgesehen, wobei der Riegelmechanismus ein Riegelelement umfasst, das durch einen elastisch deformierbaren, etwa C-förmigen Hebel gebildet wird. Durch axiale Bewegung eines Kraft-übertragungselements wird der Hebel zum Einschnappen in einen verriegelten stabilen Zustand veranlasst. US 5,626,607 A1 offenbart medizinisches Instrument mit einem langerstreckten Schaft und einem an einem distalen Endabschnitt des Schafts angeordneten Werkzeug, das zwei insbesondere zum Halten eines Gegenstands miteinander zusammenwirkende Werkzeugelemente umfasst, von denen mindestens eines mittels eines längsverschiebbaren ersten Übertragungselements bewegbar ist, wobei ein distaler Endabschnitt des ersten Übertragungselements mehrere zu einer Verschieberichtung geneigte Flächen aufweist. Ein am Schaft befestigter federbelasteter Stift mit einer komplementären schrägen Fläche greift in die geneigten Flächen des Übertragungselementes ein und hält die Werkzeugelemente in der geschlossenen Stellung, entsprechend dem Prinzip einer Ratsche.

Bei den vorgenannten Lösungen erfolgt eine Verriegelung bzw. Feststellung der Maulteile in einer Halteposition, wobei der Riegelmechanismus im distalen Bereich des Instruments angeordnet ist. Es hat sich jedoch gezeigt, dass dabei nicht immer eine ausreichende Haltekraft zum Halten insbesondere einer chirurgischen Nadel erzielt werden kann und/oder zum Verriegeln bzw. Entriegeln ein relativ hoher Kraftaufwand notwendig ist.
Es ist Aufgabe der vorliegenden Erfindung, ein medizinisches Instrument der genannten Art anzugeben, wobei die oben genannten Nachteile möglichst vermieden werden.
Diese Aufgabe wird durch ein medizinisches Instrument gemäß Anspruch 1 gelöst. Vorteilhafte Weiterbildungen der Erfindung ergeben sich aus den Unteransprüchen.
Ein erfindungsgemäßes medizinisches Instrument ist insbesondere als chirurgisches, vorzugsweise als endoskopisches Instrument ausgebildet und weist einen lang erstreckten Schaft auf, der zum Einführen in einen körperinneren Hohlraum ausgebildet ist. Der Schaft ist vorzugsweise zumindest abschnittsweise starr, kann aber auch abschnittsweise flexibel sein. Am distalen (d.h. benutzerfernen) Endabschnitt des Schafts ist ein Werkzeug angeordnet, das insbesondere zur Durchführung chirurgischer Manipulationen bei einem endoskopischen Eingriff dient. Das Werkzeug ist vorzugsweise am distalen Ende des distalen Endabschnitts des Schafts angeordnet und ist vorzugsweise derart ausgebildet, dass es zumindest in einer geschlossenen Stellung an der Spitze des Schafts mit diesem durch eine Körperöffnung in den körperinneren Hohlraum eingeführt werden kann. Das Werkzeug umfasst zwei miteinander zusammenwirkende Werkzeugelemente, die insbesondere zum Greifen und Halten eines Gegenstands ausgebildet und gegeneinander bewegbar sind. Das medizinische Instrument kann beispielsweise als Nadelhalter ausgebildet sein, wobei die Werkzeugelemente zum Greifen und Halten einer chirurgischen Nadel angepasst sind. Mindestens eines der beiden Werkzeugelemente, das im Folgenden als bewegliches Werkzeugelement bezeichnet wird, ist relativ zu dem distalen Endabschnitt des Schafts zum Zusammenwirken mit dem anderen Werkzeugelement beweglich angeordnet und mittels eines ersten Übertragungselements vom proximalen (d.h. benutzernahen) Ende des Schafts her bewegbar. Das erste Übertragungselement kann beispielsweise als Zugstange oder Schubstange ausgebildet sein und abschnittsweise innerhalb oder an einer Außenseite des Schafts verlaufen. Zum Bewegen des mindestens einen beweglichen Werkzeugelements ist das erste Übertragungselement im bzw. am Schaft längsverschiebbar gelagert. Um das mindestens eine bewegliche Werkzeugelement zu betätigen, kann am proximalen Ende des Schafts ein manuell betätigbarer Handgriff vorgesehen sein, der mit dem proximalen Ende des ersten Übertragungselements gekoppelt ist. Es kann aber auch eine motorische Betätigung des ersten Übertragungselements vorgesehen sein.

Insbesondere kann das Werkzeug ein bewegliches und ein feststehendes Werkzeugelement umfassen, wobei das bewegliche Werkzeugelement zum Greifen und Halten des Gegenstands auf das feststehende zu bewegbar ist und zum Freigeben des Gegenstands von diesem wieder fort bewegbar ist. Das bewegliche Werkzeugelement kann schwenkbar an einem Hauptteil des Werkzeugs gelagert sein und beispielsweise als Maulteil ausgebildet sein, das gegenüber einem feststehenden, starr mit dem Hauptteil verbundenen Maulteil schwenkbar ist. Das Werkzeug kann aber auch beispielsweise zwei gegeneinander bewegliche Werkzeugelemente umfassen, etwa zwei schwenkbar am Hauptteil des Werkzeugs gelagerte Maulteile, die zum Greifen und Halten des Gegenstands aufeinander zu geschwenkt und zum Freigeben des Gegenstandes voneinander fort geschwenkt werden können. Sofern zwei schwenkbare Maulteile vorgesehen sind, können diese beispielsweise auf einer gemeinsamen Achse schwenkbar gelagert sein. Das erste Übertragungselement kann in an sich bekannter Weise mit dem schwenkbaren Maulteil bzw. mit den schwenkbaren Maulteilen gekoppelt sein, so dass durch eine Längsverschiebung des ersten Übertragungselements eine Schwenkbewegung des Maulteils bzw. der Maulteile bewirkt werden kann.

Erfindungsgemäß weist ein distaler Endabschnitt des ersten Übertragungselements eine zu einer Verschieberichtung des distalen Endabschnitts des ersten Übertragungselements geneigte Fläche auf. Die Verschieberichtung ist insbesondere parallel oder näherungsweise parallel zu einer Längsachse des distalen Endabschnitts des Schafts. Weiter weist der distale Endabschnitt des Schafts oder ein mit diesem verschiebefest verbundenes Element eine ebenfalls zur Verschieberichtung des distalen Endabschnitts des ersten Übertragungsele-ments geneigte Gegenfläche auf. Die Gegenfläche kann beispielsweise an einem Außenrohr des distalen Endabschnitts des Schafts oder an einem Element des distalen Endabschnitts des Schafts angeordnet sein, das gegenüber dem Außenrohr axial nicht verschiebbar ist; das genannte Element kann beispielsweise fest mit dem Hauptteil des Werkzeugs verbunden sein. Die Fläche und die Gegenfläche, die im Folgenden auch als geneigte oder schräge Flächen bezeichnet werden, steigen insbesondere beide in proximaler oder beide in distaler Richtung an, wobei die Neigung bzw. Steigung der geneigten Flächen jeweils parallel zur Verschieberichtung betrachtet wird, beispielsweise entlang der Längsachse des distalen Endabschnitts des Schafts. Die Gegenfläche bildet mit der geneigten Fläche des distalen Endabschnitts des ersten Übertragungselements in Abhängigkeit von einer Verschiebeposition des distalen Endabschnitts des ersten Übertragungselements relativ zum distalen Endabschnitt des Schafts einen Zwischenraum aus. Insbesondere können die geneigte Fläche des distalen Endabschnitts des ersten Übertragungselements und die Gegenfläche zumindest in einer Verschiebeposition oder in einem Teilbereich eines Verschiebewegs des distalen Endabschnitts des ersten Übertragungselements einander gegenüberliegend angeordnet sein. Dabei können eine oder mehrere schräge Flächen und/oder Gegenflächen vorgesehen sein, die einander derart zugeordnet sein können, dass ein oder mehrere Zwischenräume gebildet werden.

Das erfindungsgemäße medizinische Instrument umfasst weiterhin mindestens ein Verriegelungselement, das derart in den Zwischenraum hinein bewegbar ist, dass eine Längsverschiebung des distalen Endabschnitts des ersten Übertragungselements relativ zum distalen Endabschnitt des Schafts blockiert und dadurch das mindestens eine bewegliche Werkzeugelement verriegelt werden kann. Zum Lösen der Blockierung bzw. zur Entriegelung ist das mindestens eine Verriegelungselement aus dem Zwischenraum wieder heraus bewegbar. Dabei muss das Verriegelungselement nicht vollständig in den Zwischenraum eingebracht und auch aus diesem nicht vollständig entfernt werden, sondern eine in den Zwischenraum hinein gerichtete Bewegung bzw. eine aus diesem heraus gerichtete Bewegung kann zum Blockieren bzw. zum Lösen der Blockierung ausreichen. Das mindestens eine Verriegelungselement ist insbesondere so weit in den Zwischenraum hinein bewegbar, dass es durch Kontakt mit der schrägen Fläche und der Gegenfläche eine Bewegung des ersten Übertragungselements relativ zum distalen Endabschnitt des Schafts zumindest in einer Bewegungsrichtung blockiert und dabei vorzugsweise durch Reibschluss in dem Zwischenraum gehalten wird. Entsprechend ist das mindestens eine Verriegelungselement so weit in umgekehrter Richtung bewegbar, dass der Reibschluss mit mindestens einer der den Zwischenraum bildenden schrägen Flächen aufgehoben wird und der distale Endabschnitt des ersten Übertragungselements relativ zum distalen Endabschnitt des Schafts wieder beweglich wird. Die entsprechende Bewegung des mindestens einen Verriegelungselements kann mittels eines zweiten Übertragungselements vom proximalen Ende des Schafts her steuerbar sein. Das zweite Übertragungselement kann insbesondere in einer Längsrichtung des Schafts verschiebbar sein und im Wesentlichen parallel zum ersten Übertragungselement geführt sein, etwa abschnittsweise an einer Außenseite des Schafts oder innerhalb des Schafts, und zumindest abschnittsweise als Zug- oder Schubstange ausgebildet sein. Es können mehrere Verriegelungselemente vorgesehen sein, die in der beschriebenen Weise in einen oder mehrere Zwischenräume hinein- und daraus heraus bewegbar sind.

Dadurch, dass der distale Endabschnitt des ersten Übertragungselements und der distale Endabschnitt des Schafts schräge Flächen aufweisen, die zumindest in einer Verschiebeposition des distalen Endabschnitts des ersten Übertragungselements einander gegenüberliegen, wird ein Zwischenraum gebildet, dessen Breite sich bei einer Längsverschiebung des ersten Übertragungselements relativ zum distalen Endabschnitt des Schafts verändert. Der Zwischenraum wird erfindungsgemäß genutzt, um durch Einbringen eines Verriegelungselements das erste Übertragungselement relativ zum distalen Endabschnitt des Schafts zu blockieren und damit das mindestens eine bewegliche Werkzeugelement in einer Halteposition zu arretieren, in der ein Gegenstand zwischen den Werkzeugelementen gehalten werden kann. Hierdurch kann auf einfache und sichere Weise eine Verriegelung des Werkzeugs ermöglicht werden, wodurch ein Gegenstand wie etwa eine chirurgische Nadel sicher zwischen den beiden Werkzeugelementen gehalten werden kann, ohne dass eine weitere Kraftausübung des Benutzers notwendig ist. Ferner kann hierdurch eine einfache und sichere Entriegelung zum Freigeben des gehaltenen Gegenstands ermöglicht werden, wobei zum Entriegeln vom Benutzer nur eine relativ geringe Kraft aufgebracht werden muss.

Gemäß einer bevorzugten Ausführungsform der Erfindung ist die schräge Fläche des distalen Endabschnitts des ersten Übertragungselements auf einer Außenseite des distalen Endabschnitts des ersten Übertragungselements angeordnet, und die Gegenfläche ist auf einer Innenseite eines rohrförmigen Elements angeordnet. Das rohrförmige Element kann beispielsweise ein Außenrohr des distalen Endabschnitts des Schafts sein, oder das rohrförmige Element kann das mit dem distalen Endabschnitt des Schafts verschiebefest verbundene Element sein. Insbesondere kann die Gegenfläche auf einer Innenseite eines rohr- oder hülsenförmigen Elements angeordnet sein, das drehbar, jedoch axial fixiert in das Außenrohr eingesetzt ist; das Element kann beispielsweise fest mit dem Hauptteil des Werkzeugs verbunden sein. Hierdurch wird eine einfach aufgebaute und besonders stabile und belastbare Anordnung geschaffen.

Vorzugsweise ist das Werkzeug derart ausgebildet, dass durch eine Verschiebung des ersten Übertragungselements in der proximalen Richtung die beiden Werkzeugelemente zum Greifen eines Gegenstands geschlossen werden können und somit der Gegenstand durch eine innerhalb des ersten Übertragungselements wirkende Zugspannung gehalten werden kann. Die schräge Fläche und die Gegenfläche sind dabei beide in proximaler Richtung radial ansteigend ausgebildet, d.h. in einem axialen Schnitt gesehen beispielsweise mit in proximaler Richtung zunehmendem radialen Abstand von einer Längsachse des distalen Endabschnitts des ersten Übertragungselements. Bei dieser Ausführungsform kann das mindestens eine bewegliche Werkzeugelement zum festen Halten des Gegenstands durch Erzeugen einer Zugspannung im ersten Übertragungselement mit einer Haltekraft beaufschlagt werden, das bewegliche Werkzeugelement durch Bewegen des mindestens einen Verriegelungselements in dieser Halteposition verriegelt werden und die Haltekraft durch die Zugspannung zwischen dem distalen Endabschnitt des ersten Übertragungselements und dem beweglichen Werkzeugelement aufrecht erhalten werden. Dies ermöglicht die Ausübung einer besonders hohen Haltekraft und somit ein besonders festes und sicheres Halten des gegriffenen Gegenstands.

Alternativ kann es beispielsweise vorgesehen sein, dass die Werkzeugelemente durch eine Verschiebung des ersten Übertragungselements in distaler Richtung geschlossen werden können, wobei beide schrägen Flächen in distaler Richtung radial ansteigend ausgebildet sind. Hierbei kann die Haltekraft durch eine Schubspannung zwischen dem beweglichen Werkzeugelement und dem distalen Endabschnitt des ersten Übertragungselements aufrecht erhalten werden.

In besonders bevorzugter Weise bilden die beiden die Fläche und die Gegenfläche einen in einem axialen Schnitt gesehen keilförmigen Zwischenraum aus. In dem Fall, dass die Werkzeugelemente durch Verschiebung des ersten Übertragungselements in proximaler Richtung schließbar sind und die beiden schrägen Flächen in proximaler Richtung radial ansteigen, ist der keilförmige Zwischenraum vorzugsweise nach distal spitz zulaufend und nach proximal geöffnet. Dadurch, dass der Zwischenraum keilförmig ausgebildet ist, wird es ermöglicht, den distalen Endabschnitt des ersten Übertragungselements in einer Mehrzahl von Verschiebepositionen zu blockieren und damit das mindestens eine bewegliche Maulteil in einer Mehrzahl von Positionen zu verriegeln. Hierdurch wird ein Halten von unterschiedlich groß dimensionierten Gegenständen, beispielsweise von chirurgischen Nadeln mit unterschiedlichen Durchmessern, sowie ein Ausgleich von Toleranzen ermöglicht. Ferner kann auf diese Weise ein Hinein- und Herausbewegen des mindestens einen Verriegelungselements in den bzw. aus dem Zwischenraum mit nur einem geringen Kraftaufwand und damit ein besonders einfaches Ver- und Entriegeln ermöglicht werden.

Vorzugsweise sind die schräge Fläche des distalen Endabschnitts des ersten Übertragungselements und die Gegenfläche mit solchen Neigungswinkeln zur Verschieberichtung geneigt, dass die Verschiebung des ersten Übertragungselements bei in den Zwischenraum eingeführtem Verriegelungselement selbsthemmend blockiert werden kann. Die schräge Fläche des distalen Endabschnitts des ersten Übertragungselements bildet beispielsweise einen Winkel im Bereich von beispielsweise etwa 5° bis 6° mit der Verschieberichtung des distalen Endabschnitts des ersten Übertragungselements, die zumindest näherungsweise parallel zur Längsachse des distalen Endabschnitts des Schafts verläuft, und die an dem distalen Endabschnitt des Schafts vorgesehene Gegenfläche bildet einen Winkel von beispielsweise etwa 10° bis 12° zur Verschieberichtung. Hierdurch wird eine selbsthemmende Blockierung der Bewegung des ersten Übertragungselements relativ zum distalen Endabschnitt des Schafts ermöglicht und insbesondere eine Verriegelung des mindestens einen beweglichen Werkzeugelements in beiden Richtungen, also sowohl gegen ein Öffnen als auch gegen ein weiteres Schließen der Werkzeugelemente.

In bevorzugter Weise ist das mindestens eine Verriegelungselement in einem axialen Schnitt gesehen näherungsweise keilförmig ausgebildet. Insbesondere ist das Verriegelungselement in dem Fall, dass die beiden schrägen Flächen in proximaler Richtung radial ansteigen, nach distal spitz zulaufend keilförmig ausgebildet. Hierdurch kann ebenfalls ein Halten von unterschiedlich großen Gegenständen und ein Ausgleich von Toleranzen sowie ein besonders leichtes Einführen und Herausziehen des Verriegelungselements in den bzw. aus dem Zwischenraum ermöglicht werden.

In besonders bevorzugter Weise sind sowohl der Zwischenraum als auch das Verriegelungselement in einem axialen Schnitt keilförmig ausgebildet, wobei die Keilwinkel des Verriegelungselements und des Zwischenraums im Wesentlichen gleich sind. Hierdurch kann eine größere Sicherheit bei der selbsthemmenden Blockierung erreichbar sein und zugleich eine Verriegelung in einem größeren Bereich der Längsverschiebung des ersten Übertragungselements und daher in einem größeren Bereich von Positionen des mindestens einen beweglichen Werkzeugelements bzw. Öffnungsweiten des Werkzeugs erreichbar sein. Hierdurch kann ein besonders sicheres Halten beispielsweise von Nadeln unterschiedlicher Durchmesser sowie ein verbesserter Ausgleich von Toleranzen ermöglicht werden.

Weiterhin ist es bevorzugt, dass das mindestens eine Verriegelungselement radial beweglich mit einem distalen Endabschnitt des zweiten Übertragungselements verbunden ist. Der distale Endabschnitt des zweiten Übertragungselements ist insbesondere als Verriegelungsschlitten ausgebildet, der parallel zur Verschieberichtung des distalen Endabschnitts des ersten Übertragungselements und damit zumindest näherungsweise parallel zur Längsachse des distalen Endabschnitts des Schafts verschiebbar ist. Das Verriegelungselement kann beispielsweise in radialer Richtung verschiebbar oder um eine Querachse schwenkbar mit dem Verriegelungsschlitten verbunden sein. Hierdurch wird eine einfache Anpassung des Verriegelungselements an unterschiedliche Verriegelungspositionen und damit auf einfache und sichere Weise eine Verriegelung des mindestens einen beweglichen Werkzeugelements in unterschiedlichen Stellungen ermöglicht.

Vorzugsweise trägt das mindestens eine Verriegelungselement proximalseitig zwei einander gegenüberliegende, quer zur Längsrichtung des distalen Endabschnitts des Schafts bzw. quer zur Verschieberichtung des Verriegelungsschlittens gerichtete Nasen, mit denen es am Verriegelungsschlitten angehängt bzw. in entsprechende Ausnehmungen des Verriegelungsschlittens eingehängt ist. Hierdurch wird in besonders einfacher Weise eine radiale Beweglichkeit des mindestens einen Verriegelungselements zur Anpassung an unterschiedliche Verriegelungspositionen ermöglicht.

In vorteilhafter Weise ist das mindestens eine Verriegelungselement im distalen Endabschnitt des Schafts in einer Nut geführt, deren Boden die Gegenfläche bildet. Insbesondere kann der distale Endabschnitt des Schafts rohrförmig ausgebildet sein, wobei an einer Innenseite des Rohrs oder an einer Innenseite eines in das Rohr eingesetzten rohr- oder hülsenförmigen Elements die Nut ausgebildet ist, die in der Verschieberichtung bzw. in Längsrichtung des distalen Endabschnitts des Schafts verläuft, deren Boden jedoch schräg zu dieser Richtung geneigt ist. Hierdurch wird auf besonders einfache und stabile Weise eine Verriegelung des mindestens einen beweglichen Werkzeugelements ermöglicht.

Vorzugsweise sind im distalen Endabschnitt des Schafts zwei Verriegelungselemente vorgesehen, die zur Verriegelung bzw. Entriegelung des mindestens einen beweglichen Werkzeugelements gemeinsam bewegbar sind, insbesondere mittels des zweiten Übertragungselements gemeinsam bewegbar sind, und in einen oder zwei entsprechende Zwischenräume hinein und daraus heraus bewegt werden können. Hierdurch wird auf einfache Weise eine besonders sichere Verriegelung ermöglicht.

Die schräge Fläche und/oder die Gegenfläche können näherungsweise bzw. abschnittsweise eben oder konisch oder auch in einem axialen Schnitt ballig oder hohl ausgebildet sein. Gemäß einer bevorzugten Ausführungsform sind sowohl die schräge Fläche des distalen Endabschnitts des ersten Übertragungselements als auch die dieser zugeordnete Gegenfläche des distalen Endabschnitts des Schafts näherungsweise konisch ausgebildet, wobei die jeweilige Achse des Konus zumindest näherungsweise parallel zur Verschieberichtung des distalen Endabschnitts des ersten Übertragungselements bzw. zur Längsachse des distalen Endabschnitts des Schafts gerichtet ist. In diesem Fall können zwei Verriegelungselemente vorgesehen sein, die etwa symmetrisch auf bezüglich der Längsachse einander gegenüberliegenden Seiten angeordnet sein können.

Gemäß einer weiteren bevorzugten Ausführungsform der Erfindung weist der distale Endabschnitt des ersten Übertragungselements zwei einander axial gegenüberliegende, jeweils näherungsweise ebene schräge Flächen auf, und der distale Endabschnitt des Schafts weist zwei entsprechende, an einander gegenüberliegenden Seiten angeordnete näherungsweise ebene Gegenflächen auf. In diesem Fall sind zwei axial einander gegenüberliegend angeordnete Verriegelungselemente vorgesehen, die zum Verriegeln bzw. Entriegeln in die beiden, durch die jeweiligen einander gegenüber liegenden geneigten Flächen gebildeten Zwischenräume hinein bzw. aus diesen wieder herausbewegt werden können.

Gemäß einer besonders bevorzugten Ausführungsform der Erfindung ist das Werkzeug relativ zum distalen Endabschnitt des Schafts um eine Drehachse drehbar, die mit der Längsachse des distalen Endabschnitts des Schafts zusammenfällt oder zu dieser parallel ist. Dabei kann das Werkzeug beispielsweise gegenüber einem Außenrohr des distalen Endabschnitts des Schafts drehbar sein. Weiter ist bei dieser Ausführungsform der distale Endabschnitt des ersten Übertragungselements ebenfalls um die Drehachse oder eine zu dieser parallele Achse relativ zu einem proximalen Abschnitt des ersten Übertragungselements drehbar ausgebildet. Der distale Endabschnitt des Schafts kann hierfür distalseitig ein entsprechendes Drehlager aufweisen, und der distale Endabschnitt des ersten Übertragungselements kann proximalseitig ein Drehlager aufweisen. Weiter kann ein distaler Endabschnitt des zweiten Übertragungselements als längsverschiebbarer Verriegelungsschlitten ausgebildet sein, der über ein relativ zum Verriegelungsschlitten um die Drehachse oder eine zu dieser parallele Achse drehbares Zwischenstück mit einem proximalen Abschnitt des zweiten Übertragungselements verbunden ist. Die Drehung des Werkzeugs zusammen mit dem distalen Endabschnitt des ersten Übertragungselements relativ zu den übrigen Abschnitten des Schafts bzw. des ersten Übertragungselements kann beispielsweise durch eine drehbare Welle gesteuert werden, die sich innerhalb des Schafts erstreckt und die drehfest mit einem Hauptteil des Werkzeugs verbunden ist. Dadurch, dass das Werkzeug um eine zur Längsachse des distalen Endabschnitts des Schafts parallele Drehachse drehbar ist, kann die Verwendbarkeit des medizinischen Instruments für eine Vielzahl von Situationen verbessert werden. Insbesondere kann es dadurch erreicht werden, dass die Werkzeugelemente sowie ein gegebenenfalls von diesen gehaltener Gegenstand in eine gewünschte Richtung orientiert werden können, ohne den proximalen Abschnitt des Schafts drehen zu müssen. Hierbei kann es dadurch, dass eine Verriegelung des mindestens einen beweglichen Werkzeugelements in einer Halteposition zum Halten des Gegenstands innerhalb des distalen Endabschnitts des Schafts erfolgt, ermöglicht werden, die Drehlager des Werkzeugs und des distalen Endabschnitts des ersten Übertragungselements von der Haltekraft zu entlasten. Hierdurch kann für einen Benutzer die Ausführung der Drehung erleichtert werden sowie ein übermäßiger Verschleiß des Instruments vermieden werden.

Gemäß einer bevorzugten Ausführungsform der Erfindung ist der distale Endabschnitt des Schafts relativ zu einem proximalen Abschnitt des Schafts um eine quer zur Längsachse des Schafts gerichtete Querachse schwenkbar. Die Schwenkbewegung kann beispielsweise durch ein drittes Übertragungselement, das in oder am proximalen Abschnitt des Schafts verläuft, vom proximalen Ende des Schafts her gesteuert werden. In besonderes bevorzugter Weise ist sowohl der distale Endabschnitt des Schafts um die Querachse relativ zum proximalen Abschnitt des Schafts schwenkbar als auch das Werkzeug um eine parallel zur Längsachse des distalen Endabschnitts des Schafts gerichtete Drehachse drehbar. In diesem Fall kann es vorgesehen sein, dass die drehbare Welle, die zur Übertragung der Drehbewegung des Werkzeugs dient, im Bereich eines zum Abwinkeln des distalen Endabschnitts des Schafts dienenden Schwenkgelenks unterbrochen ist und die Übertragung einer Drehung beispielsweise mittels einer Verzahnung erfolgt, über die ein proximaler Abschnitt der Welle mit einem distalen Abschnitt zur Übertragung der Drehung zusammenwirkt. Weiter kann es vorgesehen sein, dass der distale Endabschnitt des ersten Übertragungselements über ein relativ zu diesem drehbar gelagertes Zwischenstück sowie im Bereich des Schwenkgelenks des distalen Endabschnitts des Schafts über einen Verbindungshebel mit einem proximalen Abschnitt des ersten Übertragungselements verbunden ist. In entsprechender Weise kann der distale Endabschnitt des zweiten Übertragungselements über ein drehbares Zwischenstück und im Bereich des Schwenkgelenks über einen Verbindungshebel mit einem proximalen Abschnitt des zweiten Übertragungselements verbunden sein. Die Verbindungshebel können insbesondere in einem radialen Außenbereich des Schafts angeordnet sein. Die drehbare Welle zur Steuerung der Drehbewegung des Werkzeugs verläuft vorzugsweise im Inneren des Schafts, insbesondere näherungsweise koaxial mit einer jeweiligen Längsachse sowohl des proximalen Abschnitts des Schafts als auch des distalen Endabschnitts. Das Schwenkgelenk zum Schwenken des distalen Endabschnitts des Schafts, einschließlich der drehbaren Welle und des ersten Übertragungselements im Bereich des Schwenkgelenks, kann wie in den nicht vorveröffentlichten Patentanmeldungen DE 102015015664.0 und DE 102015015655.1 beschrieben ausgebildet sein, welche diesbezüglich durch Bezugnahme in die vorliegende Anmeldung aufgenommen werden. Auf diese Weise wird ein besonders vielseitig verwendbares medizinisches Instrument geschaffen, bei dem das mindestens eine bewegliche Maulteil insbesondere einer Halteposition zum Festhalten eines gegriffenen Gegenstands ohne wesentliche Belastung der Drehlager und der proximalen Abschnitte des ersten und des zweiten Übertragungselements arretiert werden kann.

Gemäß einer besonders bevorzugten Ausführungsform der Erfindung ist das mindestens eine Verriegelungselement in eine Verriegelungsposition durch Federkraft vorgespannt. Dies bedeutet beispielsweise in dem Fall, dass der Zwischenraum zwischen den einander zugeordneten schrägen Flächen in distaler Richtung keilförmig zuläuft, dass das Verriegelungselement in distaler Richtung federbelastet ist. Hierdurch kann es erreicht werden, dass das Verriegelungselement dann, wenn über das zweite Übertragungselement keine axiale Kraft ausgeübt wird, in die Verriegelungsposition gedrückt wird und somit das mindestens eine bewegliche Werkzeugelement verriegelt wird. Zum Entriegeln wird das mindestens eine Verriegelungselement mittels des zweiten Übertragungselements gegen die Federkraft bewegt, insbesondere aus dem keilförmig zulaufenden Zwischenraum so weit herausgezogen, dass der Kontakt mit mindestens einer der schrägen Flächen aufgehoben wird. Hierdurch kann die Sicherheit bei der Benutzung des medizinischen Instruments erhöht werden, da ein unbeabsichtigtes Freigeben der Bewegung des mindestens einen beweglichen Werkzeugelements mit größerer Sicherheit vermieden werden kann.

Vorzugsweise ist eine Steuerfeder, mit der das mindestens eine Verriegelungselement federbelastet ist, in einer Handhabe des medizinischen Instruments angeordnet. Hierdurch wird die Reinigung und Sterilisation des medizinischen Instruments erleichtert, da die Handhabe in der Regel nicht in direktem Kontakt mit Körperflüssigkeiten steht. Die von der Steuerfeder ausgeübte und über das zweite Übertragungselement übertragene Kraft ist relativ gering, so dass die Übertragung dieser Kraft über das zweite Übertragungselement nicht zu einer wesentlichen Belastung der Drehlager führt.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1a und 1b den distalen Endbereich eines medizinischen Instruments gemäß einem Ausführungsbeispiel der Erfindung mit geöffneten Maulteilen in einer teilweise transparenten Seitenansicht (Fig. 1a) und einer axialen Schnittansicht (Fig. 1b);
Fig. 2a und 2b den distalen Endbereich des medizinischen Instruments gemäß Fig. 1a und 1b in entsprechenden Ansichten, jedoch mit geschlossenen Maulteilen;
Fig. 3 eine Zughülse, eine Führungshülse und ein Verriegelungselement des medizinischen Instruments gemäß Fig. 1a und 1b;
Fig. 4 das medizinische Instrument gemäß Fig. 1a und 1b in einer Gesamtansicht.

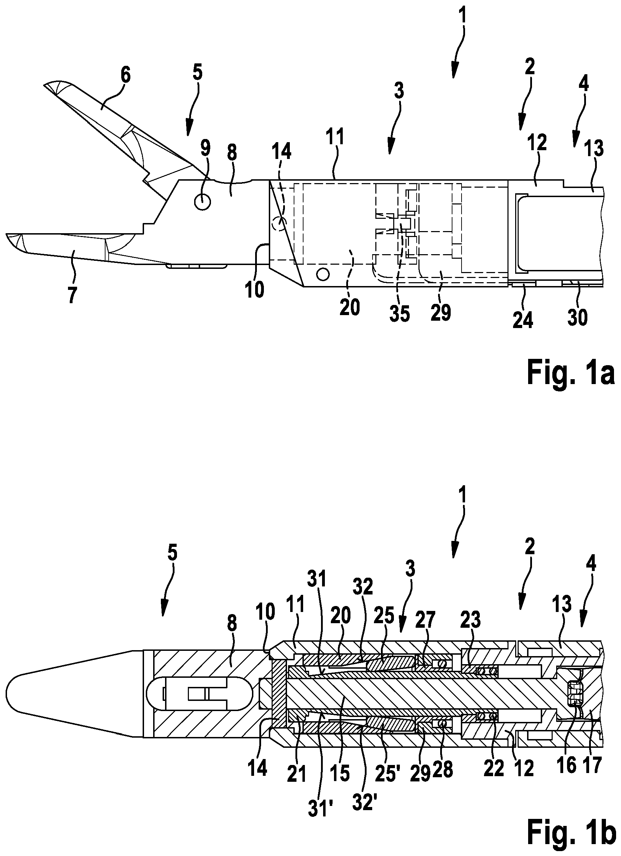

In Fig. 1a ist ein Ausführungsbeispiel eines erfindungsgemäßen medizinischen Instruments in einer teilweise aufgeschnittenen Ansicht gezeigt, wobei nur ein distaler Endbereich dargestellt ist. Das medizinische Instrument 1 umfasst einen langerstreckten, zur Einführung in eine Körperhöhle eines menschlichen oder tierischen Körpers geeigneten Schaft 2, der einen distalen Endabschnitt 3 sowie einen proximalen Abschnitt 4 umfasst, wobei von dem proximalen Abschnitt 4 des Schafts in Fig. 1a nur das distale Ende gezeigt ist. Weiter umfasst das medizinische Instrument 1 ein Werkzeug 5, das am distalen Ende des distalen Endabschnitts 3 des Schafts 2 angeordnet ist. Das Werkzeug 5 umfasst zwei Maulteile 6, 7, wovon ein erstes Maulteil 6 schwenkbar an einem Hauptteil 8 des Werkzeugs 5 gelagert ist und ein zweites Maulteil 7 starr mit dem Hauptteil 8 verbunden ist. Die Maulteile 6, 7 sind im dargestellten Ausführungsbeispiel zum Greifen und Halten einer chirurgischen Nadel ausgebildet. Auf den Innenseiten der Maulteile 6, 7 können zum sicheren Halten einer chirurgischen Nadel beispielsweise parallele Rillen angeordnet sein. Die Schwenkachse 9 des schwenkbaren Maulteils 6 verläuft quer zu einer Längsachse des distalen Endabschnitts 3 des Schafts 2. Das Hauptteil 8 ist mit einem Drehlager 10 relativ zu einem Außenrohr 11 des distalen Endabschnitts 3 des Schafts 2 um die Längsachse des distalen Endabschnitts 3 drehbar gelagert. Das Außenrohr 11 ist proximalseitig fest mit einem Gelenkteil 12 verbunden, das um eine Querachse schwenkbar am distalen Endbereich eines Außenrohrs 13 des proximalen Abschnitts 4 des Schafts 2 gelagert ist. In Fig. 1a steht die Schwenkachse, um die der distale Endabschnitt 3 gegenüber dem proximalen Abschnitt 4 des Schafts 2 schwenkbar ist, senkrecht auf der Zeichenebene.

Fig. 1b zeigt den distalen Endbereich des medizinischen Instruments 1 in einem axialen Längsschnitt, wobei die Schnittebene in Fig. la senkrecht zur Zeichenebene steht. Wie in Fig. 1b gezeigt, ist das Hauptteil 8 mit einem Stift 14 drehfest mit einem distalen Endabschnitt 15 einer drehbaren Welle verbunden. An seinem proximalen Ende ist der distale Endabschnitt 15 der drehbaren Welle über eine Verzahnung 16 mit einem proximalen Abschnitt 17 der drehbaren Welle verbunden, der sich innerhalb des proximalen Abschnitts 4 des Schafts 2 erstreckt und über den eine Drehung des Hauptteils 8 und damit der Maulteile 6, 7 um die Längsachse des distalen Endabschnitts 3 des Schafts 2 bewirkt werden kann.

Innerhalb des Außenrohrs 11 des distalen Endabschnitts 3 des Schafts 2 ist eine Führungshülse 20 angeordnet, die fest mit dem Hauptteil 8 verbunden ist und deren Funktion unten näher beschrieben wird. Innerhalb der Führungshülse 20 ist in Längsrichtung verschiebbar, jedoch gegenüber dem Hauptteil 8 und gegenüber der Führungshülse 20 drehfest, eine Zughülse 21 gelagert, die den distalen Endabschnitt 15 der drehbaren Welle aufnimmt und auf diesem gleitet. Die Zughülse 21 ist distalseitig so mit dem schwenkbaren Maulteil 6 gekoppelt, dass eine axiale Verschiebung der Zughülse 21 in distaler Richtung ein Öffnen des Maulteils 6 und eine axiale Verschiebung in proximaler Richtung ein Schließen des Maulteils 6 bewirkt. Proximalseitig ist die Zughülse 21 über ein Drehlager 22 mit einem ebenfalls axial verschiebbaren Zwischenstück 23 verbunden, das wiederum über einen Verbindungshebel 24 mit einer am bzw. im proximalen Abschnitt 4 des Schafts 2 verlaufenden Zugstange verbunden ist (s. Fig. 1a). Die Zugstange, der Verbindungshebel 24, das Zwischenstück 23, das Drehgelenk 22 und die Zughülse 21 bilden ein erstes Übertragungselement, durch dessen axiale Verschiebung die Bewegung des beweglichen Maulteils 6 zum Öffnen und Schließen des durch die Maulteile 6, 7 gebildeten Zangenmauls vom proximalen Ende des Schafts 2 her gesteuert werden kann.

In der Führungshülse 20 sind zwei symmetrisch zur Längsachse angeordnete Verriegelungselemente 25, 25' längs verschiebbar geführt. Die Verriegelungselemente 25, 25' sind mit einem ringförmigen, längsverschiebbar auf der Zughülse 21 gelagerten Verriegelungsschlitten 27 verbunden. Der Verriegelungsschlitten 27 ist über ein Drehlager 28 drehbar mit einem Zwischenstück 29 verbunden, das über einen Verbindungshebel 30 mit einer Schubstange, die am bzw. im proximalen Abschnitt 4 des Schafts 2 verläuft, verbunden ist (s. Fig. 1a). Diese Schubstange stellt zusammen mit dem Verbindungshebel 30, dem Zwischenstück 29, dem Drehlager 28 und dem Verriegelungsschlitten 27 ein zweites Übertragungselement dar, durch das eine axiale Verschiebung der Verriegelungselemente 25, 25' vom proximalen Ende des Schafts 2 aus bewirkt werden kann.

Wie in Fig. 1b erkennbar ist, weist die Zughülse 21 auf ihrer Außenseite zwei schräge Flächen 31, 31' auf, die jeweils in proximaler Richtung radial ansteigen, d.h. der Abstand von der Längsachse der Zughülse 21 bzw. des distalen Endabschnitts 3 des Schafts 2 nimmt in proximaler Richtung zu. Die Führungshülse 20 weist auf ihrer Innenseite zwei Gegenflächen 32, 32' auf, die jeweils gegenüberliegend den Flächen 31, 31' angeordnet sind. Die Gegenflächen 32, 32' steigen ebenfalls in proximaler Richtung an. Dabei ist der Neigungswinkel der Gegenflächen 32, 32' zur Längsachse größer als der Neigungswinkel der schrägen Flächen 31, 31'. In der in Fig. 1a und 1b dargestellten Position der Zughülse 21, d.h. bei geöffneten Maulteilen 6, 7, sind die schrägen Flächen 31, 31' und die Gegenflächen 32, 32' zueinander in axialer Richtung versetzt, so dass die Verriegelungselemente 25, 25' nicht zwischen die einander zugeordneten schrägen Flächen geschoben werden können. Das bewegliche Maulteil 6 kann somit durch Verschiebung des ersten Übertragungselements ungehindert geschwenkt werden.

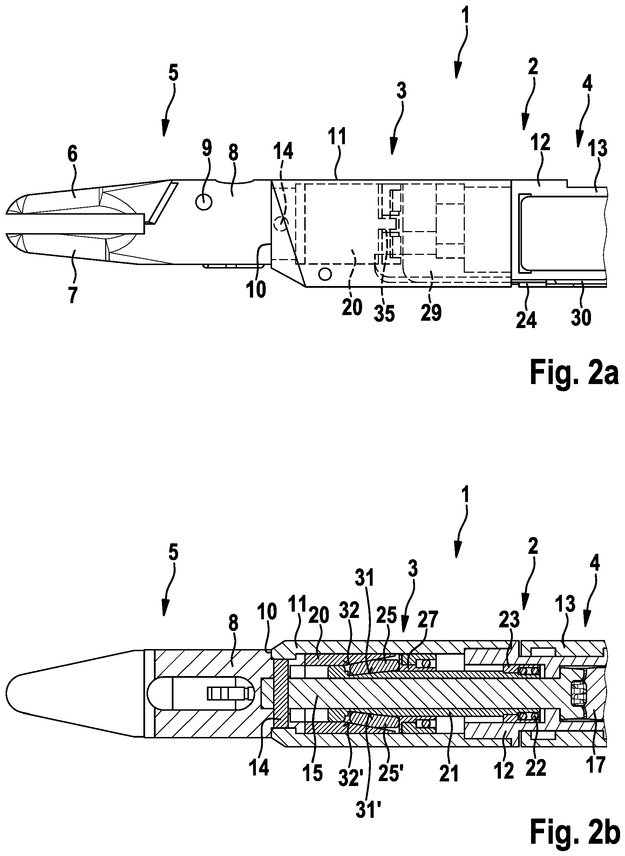

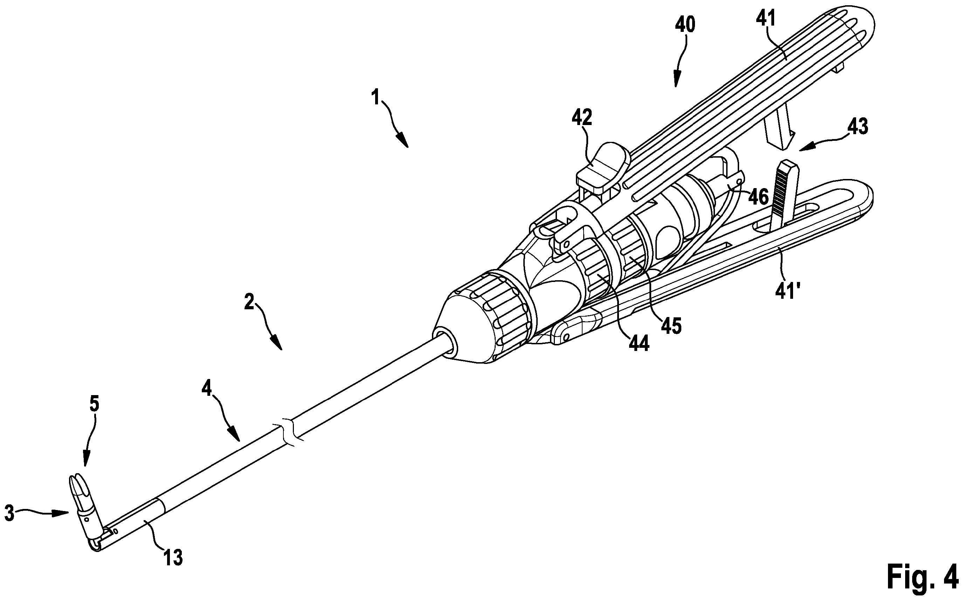

In den Figuren 2a und 2b ist der distale Bereich des medizinischen Instruments 1 wie in den Figuren 1a und 1b gezeigt, jedoch ist das Zangenmaul in Fig. 2a und 2b geschlossen. In der in den Figuren 2a und 2b gezeigten geschlossenen Position kann zwischen den Maulteilen 6, 7 beispielsweise eine chirurgische Nadel gehalten werden (nicht dargestellt). Wie insbesondere in Fig. 2b zu erkennen ist, ist die Zughülse 21 gegenüber der Position bei geöffnetem Maulteil 6 (s. Fig. 1a, 1b) in proximaler Richtung verschoben. Hierfür ist die in bzw. am proximalen Abschnitt 4 des Schafts 2 verlaufende Zugstange nach proximal gezogen worden, wodurch über den Verbindungshebel 24, das Zwischenstück 23 und das Drehlager 22 die Zughülse 21 verschoben worden ist. Aufgrund der Verschiebung der Zughülse 21 sind die schrägen Flächen 31, 31' nun jeweils derart gegenüberliegend zu den schrägen Gegenflächen 32, 32'angeordnet, dass zwischen diesen ein größerer Zwischenraum gebildet wird. Da der Neigungswinkel der Gegenflächen 32, 32' größer ist als der der schrägen Flächen 31, 31', weist der Zwischenraum in dem in Fig. 2b gezeigten axialen Längsschnitt eine keilförmige Gestalt auf. Die Zwischenräume sind bei der in Fig. 2b gezeigten Position der Zughülse 21 breit genug, um jeweils ein Verriegelungselement 25, 25' aufzunehmen. Wie im Vergleich mit Fig. 1b zu erkennen ist, ist hierfür gemäß Fig. 2b der Verriegelungsschlitten 27 in distaler Richtung verschoben worden. Dies kann durch entsprechende Betätigung der im bzw. am proximalen Abschnitt 4 des Schafts 2 verlaufenden Schubstange bewirkt werden, die über den Verbindungshebel 30, das Zwischenstück 29 und das Drehlager 28 eine entsprechende axiale Verschiebung des Verriegelungsschlittens 27 steuert. Der Verriegelungsschlitten 27 verschiebt wiederum die Verriegelungselemente 25, 25' in distaler Richtung. Die Kraft zum Verschieben der Schubstange nach distal und zum Bewegen der Verriegelungselemente 25, 25' in die Zwischenräume wird dabei durch eine in einer Handhabe des Instruments (s. Fig. 4) angeordnete Steuerfeder aufgebracht.

Die Verriegelungselemente 25, 25' weisen zumindest in ihrem distalen Bereich eine Keilform auf, wobei der Keilwinkel etwa dem Öffnungswinkel des zwischen den schrägen Flächen 31, 31' und den jeweiligen Gegenflächen 32, 32' gebildeten Zwischenraums entspricht. Wie in Fig. 2b dargestellt ist, können die Verriegelungselemente 25, 25' so weit in distaler Richtung verschoben werden, bis sie an der jeweiligen schrägen Fläche 31, 31' der Zughülse 21 und gleichzeitig an der jeweiligen Gegenfläche 32, 32' der Führungshülse 20 anliegen; hierdurch ist ein distaler Anschlag für die Bewegung der Verriegelungselemente 25, 25' erreicht. Die Verriegelungselemente 25, 25' sind dabei derart beweglich mit dem Verriegelungsschlitten 27 verbunden, dass sie beim Einschieben in die Zwischenräume den schrägen Flächen 31, 31' und den Gegenflächen 32, 32' bis zum beidseitigen Anliegen folgen können. In diesem Zustand ist eine axiale Verschiebung der Zughülse 21 in distaler Richtung nicht mehr möglich, da diese durch die Verriegelungselemente 25, 25' blockiert wird. Die Neigungswinkel der schrägen Flächen 31, 31' und der Gegenflächen 32, 32' sind derart gewählt, dass eine Selbsthemmung eintritt, wobei auch eine Verschiebung der Zughülse 21 in proximaler Richtung verhindert wird. Die schrägen Flächen 31, 31' sind hierfür um etwa 5° bis 6° gegen die Verschieberichtung der Zughülse 21 geneigt, die parallel zur Längsachse des distalen Endabschnitts 3 des Schafts 2 gerichtet ist, und die Gegenflächen 32, 32' sind um etwa 10° bis 12° gegen die Längsachse geneigt. Das bewegliche Maulteil 6 ist somit selbsthemmend arretiert, und eine zwischen den Maulteilen 6, 7 gehaltene chirurgische Nadel wird fest gehalten, ohne dass ein Benutzer hierfür weiterhin eine Kraft ausüben muss. Die Verriegelungselemente 25, 25', ebenso wie die Zughülse 21 und die Führungshülse 20, sind beispielsweise aus Edelstahl hergestellt, wobei die Verriegelungselemente 25, 25' sowie die Flächen 31, 31' und die Gegenflächen 32, 32' vorzugsweise gehärtet sind.

Zum Halten einer chirurgischen Nadel wird diese zunächst durch Schließen des Maulteils 6 gegriffen. Dabei befinden sich die Verriegelungselemente 25, 25' außerhalb der Zwischenräume zwischen den Flächen 31, 31' der Zughülse 21 und den Gegenflächen 32, 32' der Führungshülse 20. Das erste Übertragungselement wird zum Schließen des Zangenmauls in proximaler Richtung gezogen, während das zweite Übertragungselement in seiner proximalen Endposition verbleibt (s. Fig. 1a und 1b). Beim Schließen des Zangenmauls vergrößern sich die zwischen den Flächen 31, 31' und den Gegenflächen 32, 32' gebildeten Zwischenräume. Wenn die Nadel zwischen den Maulteilen 6, 7 gegriffen worden ist und sich die Maulteile 6, 7 somit in ihrer geschlossenen Stellung befinden, so wird über das erste Übertragungselement eine Haltekraft auf das bewegliche Maulteil 6 ausgeübt, um die Nadel ausreichend fest zu halten. Sodann werden mittels einer Verschiebung des zweiten Übertragungselements in distaler Richtung die Verriegelungselemente 25, 25' in die zwischen den Flächen 31, 31' und den Gegenflächen 32, 32' gebildeten Zwischenräume, die in dieser Position der Zughülse 21 ausreichend breit sind, bis zum distalen Anschlag eingeschoben (s. Fig. 2a, 2b). Wird nun das erste Übertragungselement proximalseitig losgelassen, so verklemmen sich die Verriegelungselemente 25, 25' und blockieren eine Verschiebung der Zughülse 21 relativ zur Führungshülse 20. Dadurch ist das bewegliche Maulteil 6 in seiner Position verriegelt, wobei die Zughülse 21 weiterhin unter Spannung steht und die Haltekraft auf das Maulteil 6 ausübt.

Da das Drehlager 10 des Werkzeugs entlastet ist, können die Maulteile durch Aufbringung einer Torsionskraft über die drehbare Welle 15, 17 leicht rotiert werden. Auch die Drehlager 22, 28 des ersten bzw. zweiten Übertragungselements sind entlastet und behindern die Drehung nicht. Ferner kann der distale Endabschnitt 3 des Schafts 2 ungehindert gegenüber dem proximalen Abschnitt 4 des Schafts 2 geschwenkt werden.

Zum Lösen der Verriegelung wird die Zughülse 21 über das erste Übertragungselement wieder mit Zugkraft beaufschlagt. Damit wird die Klemmkraft von den Verriegelungselementen 25, 25' genommen, die nun von der Handhabe aus gesteuert über das zweite Übertragungselement aus den Zwischenräumen herausgezogen werden können. Die Zughülse 21 kann sich bei zurückgezogenen Verriegelungselementen 25, 25' wieder frei bewegen, das Maulteil 6 kann mittels des ersten Übertragungselements geöffnet werden, und die Nadel kann aus den Maulteilen 6, 7 gelöst werden.

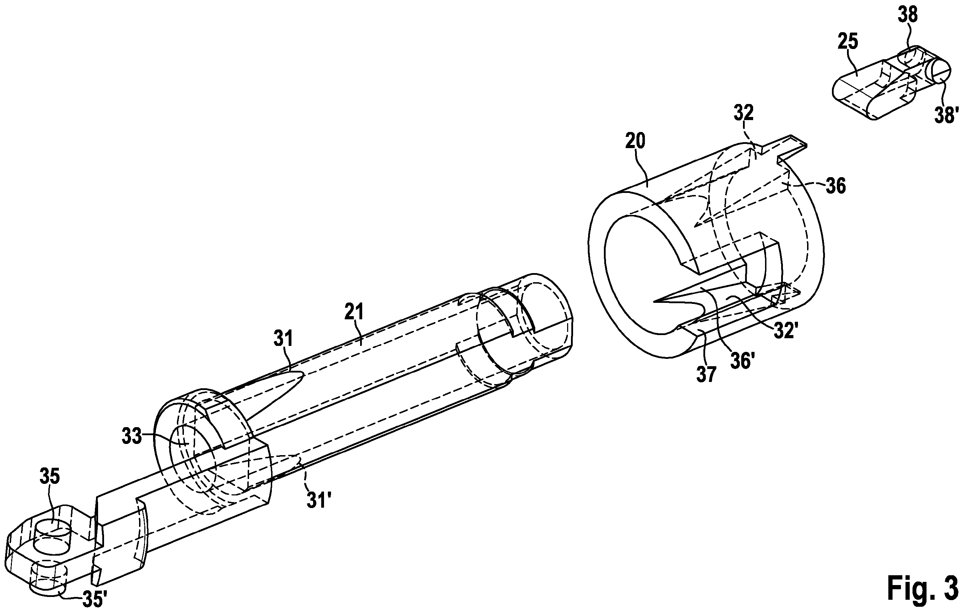

In Fig. 3 sind nach Art einer Explosionszeichnung die Zughülse 21, die Führungshülse 20 und ein Verriegelungselement 25 dargestellt. Wie in Fig. 3 gezeigt, ist die Zughülse 21 im Wesentlichen zylindrisch ausgebildet und weist einen durchgehenden inneren Hohlraum 33 zum Aufnehmen des distalen Endabschnitts 15 der drehbaren Welle auf (s. Fig. 1b). Weiter weist die Zughülse 21 einen distalen Fortsatz 34 auf, der Stifte 35, 35' trägt, die in Ausnehmungen eines gabelförmigen Fortsatzes des schwenkbaren Maulteils 6 greifen, um durch Axialverschiebung der Zughülse 21 das Maulteil 6 zu schwenken. Auf der Außenseite der Zughülse 21 sind auf gegenüber liegenden Seiten zwei ebene schräge Flächen 31, 31' angeordnet.

Die Führungshülse 20 ist im Wesentlichen als zylindrisches Rohr ausgebildet, das auf seiner Innenseite zwei einander gegenüberliegende Nuten 36, 36' trägt, in denen die Verriegelungselemente 25, 25' geführt sind. Der jeweilige Boden der Nuten 36, 36' bildet die schrägen Gegenflächen 32, 32'. Diese sind im dargestellten Ausführungsbeispiel jeweils ebene Flächen. In dem medizinischen Instrument 1 ist die Zughülse 21 innerhalb der Führungshülse 20 längsverschiebbar aufgenommen, wobei die Zughülse 21 durch den Eingriff des Fortsatzes 34 in die Ausnehmung 37 der Führungshülse 20 mit dieser drehfest verbunden ist und bei einer über den distalen Abschnitt 15 der drehbaren Welle und den Stift 14 vermittelten Drehung des Hauptteils 8 von diesem mitgedreht wird. Die schrägen Flächen 31, 31' auf der Außenseite der Zughülse 21 sind gegenüber den Nuten 36, 36' und damit gegenüber den Gegenflächen 32, 32' angeordnet.

Das Verriegelungselement 25 ist mit seinem distalen Teil in der Nut 36 aufgenommen und in dieser in Längsrichtung geführt. Der distale Teil des Verriegelungselements 25 hat eine keilförmige Gestalt, so dass das Verriegelungselement 25 leicht bis zur flächigen Berührung mit der Fläche 31 und der Gegenfläche 32 in die Nut 36 eingeführt werden kann. Proximalseitig trägt das Verriegelungselement seitlich zwei Nasen 38, 38', mit denen es am Verriegelungsschlitten 27 angehängt ist (s. Fig. 1b). Das Verriegelungselement 25 kann somit mittels des Verriegelungsschlittens 27 in axialer Richtung verschoben werden und ist dabei um die durch die Nasen 38, 38' gebildete Achse schwenkbar. Dadurch ist das Verriegelungselement 25 in radialer Richtung ausreichend beweglich, um in den zwischen der Fläche 31 und der Gegenfläche 32 gebildeten Zwischenraum bis zu dem durch Anliegen an der Fläche 31 und der Gegenfläche 32 definierten Anschlag einzudringen. Das axial gegenüber dem Verriegelungselement 25 angeordnete zweite Verriegelungselement 25' ist in gleicher Weise gestaltet und in der Nut 36' geführt.

In Fig. 4 ist das medizinische Instrument gemäß dem beschriebenen Ausführungsbeispiel in einer Gesamtansicht gezeigt. Wie in Fig. 4 dargestellt ist, kann der distale Endabschnitt 3 des Schafts 2 gegenüber dem proximalen Abschnitt 4 des Schafts 2 geschwenkt werden. Gemäß Fig. 4 ist am proximalen Ende des Schafts 2 eine Handhabe 40 angeordnet, die einen Handgriff mit zwei Griffteilen 41, 41' aufweist, die mit dem proximalen Ende der Zugstange 46 des ersten Übertragungselements verbunden sind. Durch Zusammendrücken der Griffteile 41, 41' kann die Zugstange 46 nach proximal verschoben werden und dadurch das bewegliche Maulteil 6 auf das feststehende Maulteil 7 zu bewegt werden, um etwa eine chirurgische Nadel zu greifen. An einem Griffteil 41 ist ein Schieber 42 angeordnet, der mit der Schubstange des zweiten Übertragungselements verbunden ist. Ist die Nadel fest gegriffen worden, so kann durch Verschiebung des Schiebers 42 in distaler Richtung das bewegliche Maulteil 6 entriegelt werden. Zusätzlich ist zwischen den Griffteilen 41, 41' eine Raste 43 vorgesehen. Weiterhin weist die Handhabe 40 ein Drehrad 44 auf, mit dem das Zangenmaul um eine Längsachse gedreht werden kann und das hierfür mit dem proximalen Abschnitt 17 der drehbaren Welle verbunden ist, sowie ein weiteres Drehrad 45, mit dem die Schwenkbewegung des distalen Endabschnitts 3 des Schafts 2 gesteuert werden kann.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Instrument
- 2: Schaft
- 3: Distaler Endabschnitt
- 4: Proximaler Abschnitt
- 5: Werkzeug
- 6: Maulteil
- 7: Maulteil
- 8: Hauptteil
- 9: Schwenkachse
- 10: Drehlager
- 11: Außenrohr
- 12: Gelenkteil
- 13: Außenrohr
- 14: Stift
- 15: Distaler Endabschnitt
- 16: Verzahnung
- 17: Proximaler Abschnitt
- 20: Führungshülse
- 21: Zughülse
- 22: Drehlager
- 23: Zwischenstück
- 24: Verbindungshebel
- 25, 25': Verriegelungselement
- 27: Verriegelungsschlitten
- 28: Drehlager
- 29: Zwischenstück
- 30: Verbindungshebel
- 31, 31': Fläche
- 32, 32': Gegenfläche
- 33: Hohlraum
- 34: Fortsatz
- 35, 35': Stift
- 36, 36': Nut
- 37: Ausnehmung
- 38, 38': Nase
- 40: Handhabe
- 41, 41': Griffteil
- 42: Schieber
- 43: Raste
- 44: Drehrad
- 45: Drehrad
- 46: Zugstange

## Patentansprüche

1. Medizinisches Instrument mit einem langerstreckten Schaft (2) und einem an einem distalen Endabschnitt (3) des Schafts (2) angeordneten Werkzeug (5), das zwei insbesondere zum Halten eines Gegenstands miteinander zusammenwirkende Werkzeugelemente umfasst, von denen mindestens eines mittels eines längsverschiebbaren ersten Übertragungselements bewegbar ist, **dadurch gekennzeichnet, dass** ein distaler Endabschnitt des ersten Übertragungselements eine zu einer Verschieberichtung geneigte Fläche (31, 31') aufweist und der distale Endabschnitt (3) des Schafts (2) oder ein mit diesem verschiebefest verbundenes Element eine zur Verschieberichtung geneigte Gegenfläche (32, 32') aufweist, die mit der geneigten Fläche (31, 31') einen von einer Verschiebung des distalen Endabschnitts des ersten Übertragungselements abhängigen Zwischenraum bildet, und dass mindestens ein Verriegelungselement (25, 25') vorhanden ist, das zum Blockieren der Verschiebung des ersten Übertragungselements in den Zwischenraum hinein gerichtet und zum Lösen der Blockierung aus diesem heraus gerichtet bewegbar ist.

2. Medizinisches Instrument nach Anspruch 1, **dadurch gekennzeichnet, dass** die geneigte Fläche (31, 31') auf einer Außenseite des distalen Endabschnitts des ersten Übertragungselements und die Gegenfläche (32, 32') auf einer Innenseite eines rohrförmigen Elements angeordnet ist.

3. Medizinisches Instrument nach Anspruch 1 oder 2, **dadurch gekennzeichnet, dass** die miteinander zusammenwirkenden Werkzeugelemente durch eine Verschiebung des ersten Übertragungselements in eine proximale Richtung schließbar sind und dass die geneigte Fläche (31, 31') und die Gegenfläche (32, 32') in proximaler Richtung radial ansteigend sind.

4. Medizinisches Instrument nach einem der vorhergehenden Ansprüche, **dadurch gekennzeichnet, dass** der Zwischenraum keilförmig ausgebildet ist.

5. Medizinisches Instrument nach einem der vorhergehenden Ansprüche, **dadurch gekennzeichnet, dass** die geneigte Fläche (31, 31') und die Gegenfläche (32, 32') in einem solchen Winkel zur Verschieberichtung geneigt sind, dass die Verschiebung des ersten Übertragungselements selbsthemmend blockiert werden kann.

6. Medizinisches Instrument nach einem der vorhergehenden Ansprüche, **dadurch gekennzeichnet, dass** das mindestens eine Verriegelungselement (25, 25') keilförmig ausgebildet ist.

7. Medizinisches Instrument nach einem der vorhergehenden Ansprüche, **dadurch gekennzeichnet, dass** das mindestens eine Verriegelungselement (25, 25') radial beweglich mit einem distalen Endabschnitt eines zweiten Übertragungselements verbunden ist.

8. Medizinisches Instrument nach Anspruch 7, **dadurch gekennzeichnet, dass** das mindestens eine Verriegelungselement (25, 25') mit zwei Nasen (38, 38') am distalen Endabschnitt des zweiten Übertragungselements angehängt ist.

9. Medizinisches Instrument nach einem der vorhergehenden Ansprüche, **dadurch gekennzeichnet, dass** das mindestens eine Verriegelungselement (25, 25') in einer Nut (36, 36') des distalen Endabschnitts (3) des Schafts (2) verschiebbar geführt ist, wobei ein Boden der Nut (36, 36') die Gegenfläche (32, 32') bildet.

10. Medizinisches Instrument nach einem der vorhergehenden Ansprüche, **dadurch gekennzeichnet, dass** zwei Verriegelungselemente (25, 25') vorhanden sind, die gemeinsam bewegbar sind.

11. Medizinisches Instrument nach einem der vorhergehenden Ansprüche, **dadurch gekennzeichnet, dass** das Werkzeug (5) um eine zu einer Längsachse des distalen Endabschnitts (3) des Schafts (2) parallele Drehachse drehbar ist und der distale Endabschnitt des ersten Übertragungselements relativ zu einem proximalen Abschnitt um die Drehachse drehbar gelagert ist.

12. Medizinisches Instrument nach einem der vorhergehenden Ansprüche, **dadurch gekennzeichnet, dass** der distale Endabschnitt (3) des Schafts (2) relativ zu einem proximalen Abschnitt (4) des Schafts (2) um eine Querachse schwenkbar ist.

13. Medizinisches Instrument nach einem der vorhergehenden Ansprüche, **dadurch gekennzeichnet, dass** das mindestens eine Verriegelungselement (25, 25') in eine Verriegelungsposition federbelastet ist.

14. Medizinisches Instrument nach Anspruch 13, **dadurch gekennzeichnet, dass** eine Steuerfeder zur Federbelastung des mindestens einen Verriegelungselements in einer Handhabe (40) des medizinischen Instruments angeordnet ist.

## Claims

1. Medical instrument having an elongate shaft (2) and a tool (5) arranged at a distal end portion (3) of the shaft (2), said tool comprising two tool elements which, in particular, interact with one another for holding an object, at least one of said tool elements being movable by means of a longitudinally displaceable first transmission element, **characterized in that** a distal end portion of the first transmission element has a face (31, 31') that is inclined to a displacement direction and the distal end portion (3) of the shaft (2) or an element connected to the latter in a non-displaceable manner has a counterface (32, 32') that is inclined to the displacement direction, said counterface, together with the inclined face (31, 31'), forming an interstice that depends on a displacement of the distal end portion of the first transmission element, and **in that** at least one locking element (25, 25') is present, which is movable directed into the interstice for blocking the displacement of the first transmission element and which is movable directed out of said interstice for releasing the block.

2. Medical instrument according to Claim 1, **characterized in that** the inclined face (31, 31') is arranged on an outer side of the distal end portion of the first transmission element and the counterface (32, 32') is arranged on an inner side of a tube-shaped element.

3. Medical instrument according to Claim 1 or 2, **characterized in that** the tool elements interacting with one another are closable by a displacement of the first transmission element in a proximal direction and **in that** the inclined face (31, 31') and the counterface (32, 32') are radially increasing in the proximal direction.

4. Medical instrument according to any one of the preceding claims, **characterized in that** the interstice has a wedge-shaped embodiment.

5. Medical instrument according to any one of the preceding claims, **characterized in that** the inclined face (31, 31') and the counterface (32, 32') are inclined at such an angle to the displacement direction that the displacement of the first transmission element can be blocked in self-retaining fashion.

6. Medical instrument according to any one of the preceding claims, **characterized in that** the at least one locking element (25, 25') has a wedge-shaped embodiment.

7. Medical instrument according to any one of the preceding claims, **characterized in that** the at least one locking element (25, 25') is connected in radially movable fashion to a distal end portion of a second transmission element.

8. Medical instrument according to Claim 7, **characterized in that** the at least one locking element (25, 25') is attached with two lugs (38, 38') at the distal end portion of the second transmission element.

9. Medical instrument according to any one of the preceding claims, **characterized in that** the at least one locking element (25, 25') is displaceably guided in a groove (36, 36') of the distal end portion (3) of the shaft (2), wherein a base of the groove (36, 36') forms the counterface (32, 32').

10. Medical instrument according to any one of the preceding claims, **characterized in that** two locking elements (25, 25') are present, said locking elements being movable together.

11. Medical instrument according to any one of the preceding claims, **characterized in that** the tool (5) is rotatable about an axis of rotation that is parallel to a longitudinal axis of the distal end portion (3) of the shaft (2) and the distal end portion of the first transmission element is mounted so as to be rotatable about the axis of rotation relative to a proximal portion.

12. Medical instrument according to any one of the preceding claims, **characterized in that** the distal end portion (3) of the shaft (2) is pivotable about a transverse axis relative to a proximal portion (4) of the shaft (2).

13. Medical instrument according to any one of the preceding claims, **characterized in that** the at least one locking element (25, 25') is springloaded in a locking position.

14. Medical instrument according to Claim 13, **characterized in that** a control spring for applying a spring load to the at least one locking element is arranged in a handle (40) of the medical instrument.

## Revendications

1. Instrument médical avec une tige allongée (2) et un outil (5) disposé à une partie d'extrémité distale (3) de la tige (2), qui comprend deux éléments d'outil coopérant l'un avec l'autre en particulier pour maintenir un objet, et dont au moins un est mobile au moyen d'un premier élément de transmission déplaçable longitudinalement, **caractérisé en ce qu'**une partie d'extrémité distale du premier élément de transmission présente une face (31, 31') inclinée par rapport à une direction de déplacement et la partie d'extrémité distale (3) de la tige (2) ou un élément solidaire en mouvement de celle-ci présente une face opposée (32, 32') inclinée par rapport à la direction de déplacement, qui forme avec la face inclinée (31, 31') un espace intermédiaire dépendant d'un déplacement de la partie d'extrémité distale du premier élément de transmission, et **en ce qu'**il se trouve au moins un élément de verrouillage (25, 25'), qui est déplaçable dans l'espace intermédiaire pour le blocage du déplacement du premier élément de transmission et hors de celui-ci pour lever le blocage.

2. Instrument médical selon la revendication 1, **caractérisé en ce que** la face inclinée (31, 31') est disposée sur un côté extérieur de la partie d'extrémité distale du premier élément de transmission et la face opposée (32, 32') est disposée sur un côté intérieur d'un élément tubulaire.

3. Instrument médical selon la revendication 1 ou 2, **caractérisé en ce que** les éléments d'outil coopérant l'un avec l'autre peuvent être fermés par un déplacement du premier élément de transmission dans une direction proximale et **en ce que** la face inclinée (31, 31') et la face opposée (32, 32') sont radialement ascendantes en direction proximale.

4. Instrument médical selon l'une quelconque des revendications précédentes, **caractérisé en ce que** l'espace intermédiaire est réalisé en forme de coin.

5. Instrument médical selon l'une quelconque des revendications précédentes, **caractérisé en ce que** la face inclinée (31, 31') et la face opposée (32, 32') sont inclinées par rapport à la direction de déplacement d'un angle tel que le déplacement du premier élément de transmission puisse être bloqué par auto-blocage.

6. Instrument médical selon l'une quelconque des revendications précédentes, **caractérisé en ce que** ledit au moins un élément de verrouillage (25, 25') est réalisé en forme de coin.

7. Instrument médical selon l'une quelconque des revendications précédentes, **caractérisé en ce que** ledit au moins un élément de verrouillage (25, 25') est assemblé de façon déplaçable radialement à une partie d'extrémité distale d'un deuxième élément de transmission.

8. Instrument médical selon la revendication 7, **caractérisé en ce que** ledit au moins un élément de verrouillage (25, 25') est suspendu avec deux ergots (38, 38') à la partie d'extrémité distale du deuxième élément de transmission.

9. Instrument médical selon l'une quelconque des revendications précédentes, **caractérisé en ce que** ledit au moins un élément de verrouillage (25, 25') est guidé de façon déplaçable dans une rainure (36, 36') de la partie d'extrémité distale (3) de la tige (2), dans lequel un fond de la rainure (36, 36') forme la face opposée (32, 32').

10. Instrument médical selon l'une quelconque des revendications précédentes, **caractérisé en ce qu'**il se trouve deux éléments de verrouillage (25, 25'), qui sont déplaçables ensemble.

11. Instrument médical selon l'une quelconque des revendications précédentes, **caractérisé en ce que** l'outil (5) peut tourner autour d'un axe de rotation parallèle à un axe longitudinal de la partie d'extrémité distale (3) de la tige (2) et la partie d'extrémité distale du premier élément de transmission est montée de façon rotative autour de l'axe de rotation par rapport à une partie proximale.

12. Instrument médical selon l'une quelconque des revendications précédentes, **caractérisé en ce que** la partie d'extrémité distale (3) de la tige (2) peut pivoter autour d'un axe transversal par rapport à une partie proximale (4) de la tige (2).

13. Instrument médical selon l'une quelconque des revendications précédentes, **caractérisé en ce que** ledit au moins un élément de verrouillage (25, 25') est chargé par un ressort dans une position de verrouillage.

14. Instrument médical selon la revendication 13, **caractérisé en ce qu'**un ressort de commande pour la mise en charge élastique dudit au moins un élément de verrouillage est disposé dans une poignée (40) de l'instrument médical.
